# EUROPEAN PATENT SPECIFICATION

(11) **EP 3 954 356 B1**
(45) Date of publication and mention of the grant of the patent: **02.07.2025**
(21) Application number: 19904271.4
(22) Date of filing: 17.12.2019
(51) Int. Cl.: A61K 8/365, A61K 8/44, A61K 8/9706, A61Q 5/02, A61Q 5/12

(54) **COSMETIC HAIR CARE PRODUCT**
KOSMETISCHES PRODUKT ZUR HAARPFLEGE
COMPOSITION COSMÉTIQUE POUR SOINS CAPILLAIRES

(30) Priority: 24.05.2019 RU 2018147576; 24.05.2019 RU 2019110500
(43) Date of publication of application: 16.02.2022
(73) Proprietor: Obshchestvo S Ogranichennoy Otvetstvennostyu "Splat Global", Novgorodskaya Oblast', 174350 (RU)
(72) Inventor: BELOUS, Elena Yur'evna, Moskva, 121309 (RU)
(74) Representative: Glawe, Delfs, Moll
(86) International application number: PCT/RU2019/000914
(87) International publication number: WO 2020/139145

(56) References cited:
- DATABASE GNPD [online] MINTEL; 12 January 2018 (2018-01-12), ANONYMOUS: "Shampoo", XP055723302, retrieved from www.gnpd.com Database accession no. 5375121
- DATABASE GNPD [online] MINTEL; 11 July 2013 (2013-07-11), ANONYMOUS: "Volumizing Shampoo", XP055723280, retrieved from www.gnpd.com Database accession no. 2116831
- DATABASE GNPD [online] MINTEL; 4 December 2018 (2018-12-04), ANONYMOUS: "Hair Serum", XP055677097, retrieved from www.gnpd.com Database accession no. 6174209
- ANONYMOUS: "Permanent Hair Coloring Kit", GNPD, MINTEL, 1 June 2009 (2009-06-01), XP002728010

## Description

### FIELD OF THE INVENTION

The invention relates to the field of cosmetology and can be used for the manufacture of hair care products.

The function of the hair-covering of a modem person is not only protecting the scalp, but also ensuring the perception of physical attractiveness. Hair loss, discoloration, the appearance of dandruff and other emerging problems with hair and scalp cause not only aesthetic discomfort, but can also indicate improper hair care or even presence of some diseases [1-2]. Attractive appearance is associated with the concept of "healthy hair", which can be formulated as follows: shiny and voluminous hair with a soft texture, uncut ends, noticeably moistened along the entire length, and resistant to mechanical stress [3]. All these indicators are determined by structure of the hair.

Morphologically, human hair is divided into three components: cortex, cuticle and medulla [4-6]. Cortex occupies a large part (~75%) of the hair fiber; it consists of α-keratin and a lipid cell-membrane complex. Long chains of α-keratin form a strong, but at the same time flexible, mobile structure that provides elasticity and flexibility of the hair [3]. In turn, keratin consists of 19 amino acids, among which there is no oxyproline and oxylysine, the main amino acids of collagen, and instead of them there are sulfur-containing amino acids cystine and cysteine [8-10]. The three main types of bonds that can be noted inside the hair fiber are hydrogen, disulfide, and ionic (salt bridges) bonds [5, 11].

It is important to understand that the higher the concentration of cystine in the hair structure, the more disulfide bonds are formed, which result in an increase in the strength of the hair and its resistance to external damage [11]. Hydrogen and ionic bonds are directly affected by the amount of water in the hair, which it is able to absorb from the environment and cosmetics. That is why the hair tends to change its physical characteristics depending on the humidity of the environment.

The cuticle has a layered structure and consists of keratin scales glued together by lipids (skin lipid barrier). It includes four layers, differing in chemical composition and reactivity. The A layer and exocuticle contain the largest amount of cysteine (30% and 15% by weight, respectively) and have hydrophobic properties [10,12]. An endocuticle (3% of cysteine) consists of non-keratinous material, such as enzymes, vitamins, nucleic acids, carbohydrates and fatty acids, which are mostly water-soluble, as a result of which the endocuticle exhibits hydrophilic properties [12, 13]. The epicuticle is the most significant layer in the cuticle composition. This is a hydrophobic layer of protein nature (12% of cysteine), 2.5 nm thick [13]. The cuticle layers are interconnected by a cellular membrane complex, which is a hydrophilic material rich in polar amino acids, including lysine [14, 15]. It is believed that substances can diffuse into human hair through the layer of the endocuticle and the cell membrane complex. [10].

Medulla is a cylindrical layer in the very center of the hair containing a high concentration of lipids and a small amount of cystine. Cosmetics do not affect this layer [16].

As the hair grows, it undergoes various types of influences: 1) mechanical (frequent and rough combing, backcombing, tight pulling, twisting on curlers); 2) thermal (hair drying, curling); 3) chemical (perm, hair coloring); 4) ecological, i.e. environmental impact (low-quality water, toxins, UV radiation) [17]. As a result of all types of impacts, the key hair building blocks (proteins and lipids) are degraded, which leads to visible consequences, such as cut ends, dullness, dryness, brittleness, lack of smoothness and volume of hair, increase in waviness of hair, etc.

### BACKGROUND OF THE INVENTION

Currently, the requirements for hair care products are increasing. However, if the hair is damaged, neither physical nor chemical effects are able to bring it to its original state [7]. That is why the effect of cosmetics should be aimed at preventing unwanted impact on the hair, for example, creating an additional protein film on the hair surface, which can protect the cuticle from external attacks. These effects can be achieved by adding various highly active components and complexes based on them to shampoos.

The prior art discloses a commercially available well-reputed SPLAT Heya^{™} Luxury Hair Care Bio Shampoo (Russia), which has the following composition: Water, Sodium Coco-sulfate, Cocamidopropyl Betaine, Decyl Glucoside, Sodium Lauroyl Sarcosinate, Sodium Cocoamphoacetate, Sodium Chloride, Hydroxypropyltrimonium Hydrolyzed Wheat Protein, Panthenol, Dicaprylyl Ether, Glyceryl Oleate, Glycerin, *Aloe barbadensis* Leaf Extract, Benzyl Alcohol, Phytantriol, Parfum, Sodium PCA, Sodium Lactate, *Cystoseira compressa* Extract, Hydrolyzed *ceratonia siliqua* Seed Extract, *Crithmum maritimum* Extract, *Himanthalia elongata* Extract, *Laminaria digitata* Extract, *Laminaria saccharina* Extract, *Lithothamnium calcareum* Extract, *Palmaria palmata* Extract, *Porphyra umbilicalis* Extract, *Spirulina maxima* Extract, Arginine, Aspartic Acid, PCA, Glycine, Alanine, Serine, Valine, Proline, Threonine, Isoleucine, Histidine, Phenylalanine, *Lavandula officinalis* Flower Oil, Hydroxypropylguar Hydroxypropyltrimonium Chloride, *Zea mays* Starch, Gluconolactone, Guar hydroxypropyltrimonium chloride, Polyquaternium-10, Polyquaternium-7, Citric Acid, Lactic Acid, Benzoic Acid, Dehydroacetic Acid, Phenoxyethanol, Sodium Benzoate, Potassium Sorbate, Calcium Gluconate, Tetrasodium Glutamate Diacetate, Linalool (INCI names: Aqua, Sodium coco-sulfate, Cocamidopropyl betaine, Decyl glucoside, Sodium lauroyl sarcosinate, Sodium cocoamphoacetate, Sodium chloride, Hydroxypropyltrimonium hydrolyzed wheat protein, Panthenol, Dicaprylyl ether, Glyceryl oleate, Glycerin, Aloe barbadensis leaf extract, Benzyl alcohol, Phytantriol, Parfum, Sodium PCA, Sodium lactate, Cystoseira compressa extract, Hydrolyzed ceratonia siliqua seed extract, Crithmum maritimum extract, Himanthalia elongata extract, Laminaria digitata extract, Laminaria saccharina extract, Lithothamnium calcareum extract, Palmaria palmata extract, Porphyra umbilicalis extract, Spirulina maxima extract, Arginine, Aspartic acid, PCA, Glycine, Alanine, Serine, Valine, Proline, Threonine, Isoleucine, Histidine, Phenylalanine, Lavandula officinalis flower oil, Hydroxypropyl guar hydroxypropyltrimonium chloride, Zea mays starch, Gluconolactone, Guar hydroxypropyltrimonium chloride, Polyquaternium-10, Polyquaternium-7, Citric acid, Lactic acid, Benzoic acid, Dehydroacetic acid, Phenoxyethanol, Sodium benzoate, Potassium sorbate, Calcium gluconate, Tetrasodium glutamate diacetate, Linalool).

The saturated base composition of this shampoo is a cocktail of more than 7 algae, a complex of 11 amino acids, wheat proteins, lavender essential oil, carob tree biofunctional peptides, cystozira extract, and D-Panthenol. Biofunctional carob peptides and cystozira extract in combination with D-Panthenol work from the inside, restore the integrity of the hair fibers, thicken them and nourish the scalp. Aloe Vera gel intensively moisturizes hair, refreshes and saturates it with vitamins and minerals. Lavender essential oil improves microcirculation of the scalp and helps restore balance and harmony.

Extensive clinical trials have confirmed the increase in volume and thickening of hair, increase in strength and resistibility of hair, improve the structure and quality of hair, facilitate combing, which is achieved due to the multicomponent composition of the specified shampoo.

The prior art discloses a commercially available well-reputed SPLAT Heya^{™} Luxury Hair Care Bio Balsam (Russia), which has the following composition: Water, Glycerin, *Laminaria saccharina* Extract, *Cystoseira compressa* Extract, *Fucus vesiculosus* Extract, *Chlorella vulgaris* Extract, *Chondrus crispus* Extract, Cetearyl Alcohol, Behenamidopropyl Dimethylamine, Octyldodecanol, Hydroxypropyltrimonium Hydrolyzed Wheat Protein, *Simmondsia chinensis* Seed Oil, Maltooligosyl Glucoside, Hydrogenated Starch Hydrolysate, Lactic Acid, Hydroxyethylcellulose, Cetrimonium Bromide, Rice Bran Wax, Phospholipids, *Glycine soja* Oil, Glycolipids, *Glycine soja* Sterols, Benzyl Alcohol, *Bertholletia excelsa* Seed Oil, Parfum, Sodium PCA, Sodium Lactate, *Lyceum barbarum* Fruit Extract, *Styrax tonkinensis* Resin Extract, *Vanilla planifolia* Bean Extract, *Crithmum maritimum* Extract, *Himanthalia elongate* Extract, *Laminaria digitata* Extract, *Lithothamnium calcareum* Extract, *Palmaria palmate* Extract, *Porphyra umbilicalis* Extract, *Spirulina maxima* Extract, Arginine, Aspartic Acid, PCA, *Bulnesia sarmientoi* Extract, *Citrus nobilis* (mandarin) Oil, Glycine, Alanine, Serine, Valine, Proline, Threonine, Isoleucine, Histidine, Phenylalanine, Benzoic Acid, Dehydroacetic Acid, Tetrasodium Glutamate Diacetate, Phenoxyethanol, Sodium Benzoate, Potassium Sorbate, Sorbic Acid, Citric Acid, Limonene (INCI names: Aqua, Glycerin, Laminaria saccharina extract, Cystoseira compressa extract, Fucus vesiculosus extract, Chlorella vulgaris extract, Chondrus crispus extract, Cetearyl alcohol, Behenamidopropyl dimethylamine, Octyldodecanol, Hydroxypropyltrimonium hydrolyzed wheat protein, Simmondsia chinensis seed oil, Maltooligosyl glucoside, Hydrogenated starch hydrolysate, Lactic acid, Hydroxyethylcellulose, Cetrimonium bromide, Oryza sativa cera, Phospholipids, Glycine soja oil, Glycolipids, Glycine soja sterols, Benzyl alcohol, Bertholletia excelsa seed oil, Parfum, Sodium PCA, Sodium lactate, Lyceum barbarum fruit extract, Styrax tonkinensis resin extract, Vanilla planifolia bean extract, Crithmum maritimum extract, Himanthalia elongate extract, Laminaria digitata extract, Lithothamnium calcareum extract, Palmaria palmate extract, Porphyra umbilicalis extract, Spirulina maxima extract, Arginine, Aspartic acid, PCA, Bulnesia sarmientoi extract, Citrus nobilis (mandarin) oil, Glycine, Alanine, Serine, Valine, Proline, Threonine, Isoleucine, Histidine, Phenylalanine, Benzoic acid, Dehydroacetic acid, Tetrasodium glutamate diacetate, Phenoxyethanol, Sodium benzoate, Potassium sorbate, Sorbic acid, Citric acid, Limonene).

The saturated base composition of this balm is a cocktail of more than 7 algae, a complex of 11 amino acids, wheat proteins, lavender essential oil, carob tree biofunctional peptides, cystozira extract, and D-Panthenol. Biofunctional carob peptides and cystozira extract in combination with D-Panthenol work from inside, restore the integrity of the hair fibers, thicken them and nourish the scalp. Aloe Vera gel intensively moisturizes hair, refreshes and saturates it with vitamins and minerals. Avocado oil and jojoba oil improve the texture of the hair and protect them from adverse effects. Lavender essential oil improves microcirculation of the scalp and helps restore balance and harmony.

Extensive clinical trials have confirmed the increase in volume and thickening of hair, increase in strength and resistibility of hair, improve structure and quality of hair, facilitate combing, which is achieved due to the multicomponent composition of the specified balm.

Although there are currently many balms and shampoos on the market, there remains a need to provide alternative complexes based on other cosmetic substances that would make a significant contribution in this area, expanding the range of cosmetics. This is important because use of cosmetics by the consumer requires an individual approach. While some cosmetics are acceptable for some consumers, they may be unsuitable for some other consumers for various reasons, including allergies. Therefore, in order to satisfy the needs of the widest possible range of consumers, the search and creation of such alternative means is important. The authors of the present invention conducted many studies to establish alternative combinations that would increase the strength of the hair, as expected, by reducing the porosity of the hair while increasing the thickness of the hair, and provide an increase in the density of hair growth, their elasticity and gloss.

The prior art discloses numerous complexes based on plant, protein, mineral, extraction or other raw materials for hair care, which have a stimulating effect on the scalp and prevent hair loss.

Mintel GNPD abstract 5375121 "Shampoo", January 2018 shows a shampoo formulation comprising magnesium aspartate, zinc gluconate, copper gluconate and trimethylglycine (betaine).

Protein-amino acid complexes are widespread, which include proteins, mainly based on plant materials, in particular grain (corn, wheat, etc.), and amino acids obtained from natural substances or artificial ones.

Thus, patent RU2491913C1 (published on 10.09.2013) discloses a cosmetic composition for the manufacture of hair care products containing amino acids, proteins, saccharides, vitamins, functional additives and water, differing in that it additionally contains shark liver oil with the following component ratio, wt%: Amino Acids 0.06-5.5, Proteins 0.05-1.0, Saccharides 0.05-2.0, Vitamins 0.03-15.0, Shark Liver Oil 0.5- 4.0, Functional Additives 4.0-20.0, Water - the rest. According to the authors, the invention provides a significant reduction in hair loss.

The well-known moisturizing Rice aminos & wheat protein intensive moisture shampoo of the Australian organic brand A'KIN^{™} contains a protein-amino acid complex that intensively moisturizes, nourishes, restores the hair structure due to the content of rice amino acids, wheat proteins, vitamins E and B5. Allegedly, using the shampoo will make the hair shiny, supple and elastic (RICE AMINOS & WHEAT PROTEIN INTENSIVE MOISTURE SHAMPOO A'KIN <URL: https://bling-blings.ru/items/2224-shampun-uvlazhnyayuschiy-risovye-aminokisloty-i-proteiny-pshenitsy-a-kin>).

The prior art discloses Umi No Uruoi Sou rinse shampoo of Japanese brand Kracie^{™}, containing a protein-amino acid complex of more than a dozen extracts of red, green and brown algae containing a large percentage of amino acids and proteins. The rinse shampoo is alleged to nourish the hair with all the necessary elements and maintain its gloss, elasticity and radiance all day long. Kracie Umi No Uruoi Sou revitalizing shampoo-conditioner 2 in 1 with seaweed extracts and pearl protein 520 ml <URL: http://www.esky.ru/soap/product/shampun-opolaskivatel-kracie-umi-no-uruoi-sou-2-v-1-vosstanavlivayuschiy-s-ekstraktami-morskih-vodorosley-i-proteinom-zhemchuga-520-ml-694088/>).

The disadvantage of the above complexes for hair care is that all the products described are designed to solve certain identified problems (hair loss, hair nutrition, hair moisturizing). In addition, the disclosures described generally provide information about changes in the appearance of the hair and sensory characteristics, but do not provide information on a qualitative change in the structure of the hair - an increase in the thickness of the shaft, cross-sectional area, or decrease in porosity. Also, some components of extracts and concentrates that were used in the described sources of information cannot penetrate into the hair due to their size, therefore they can act only on the surface, improving the appearance of the hair, but they do not affect the structure of the hair.

An invention has been proposed (see RU2631620C1 published on September 25, 2017), that ensures comprehensive care for the scalp and hair due to the external action and penetration of the active components into the hair and scalp. The issue to be solved was increasing the strength of hair by reducing porosity and increasing thickness of the hair shaft, as well as increasing the density of hair growth. The proposed protein-amino acid complex for hair care consists of components A, B and C, where component A is a mixture of the following amino acids: arginine, aspartic acid, pyrrolidone carboxylic acid, glycine, alanine, serine, valine, proline, threonine, isoleucine, histidine, phenylalanine, component B is a mixture containing extracts of brown and red algae, where extracts of brown algae are a mixture of *Himanthalia Elongata* extract, *Laminaria Digitata* extract and *Laminaria Saccharina* extract, extracts of red algae are a mixture of *Lithothamnium Calcareum* extract, *Palmaria Palmata* extract and *Porphyra Umbilicalis* extract, spirulina extract and *Crithmum maritimum* extract, and component B is a hydrolyzed wheat protein, in the following ratio of A 30.0-48.0, B 3.5-6.5, C - the rest, wt.%.

As a result of the studies, it was found that the complex according to RU2631620C1 with the claimed ratio of the components allows an increase in strength of the hair, as expected, by reducing porosity of the hair while increasing thickness of the hair. In addition, when using the complex, an increase in the density of hair growth is ensured. The mechanism of action of the claimed complex on the hair is not completely understood and studied, however, in the opinion of the authors of the said patent, this is due to the synergistic effect of biologically active substances from extracts of specifically selected brown and red algae with extracts of *Crithmum maritimum* and spirulina, hydrolyzed wheat protein, as well as interchangeable and essential amino acids to most accurately imitate the amino acid composition of the hair. The ratio of the complex components was chosen for the reason that when going beyond the claimed limits of the component content, the technical result is not achieved or is achieved to a minor extent. As to the content of brown or red algae in the complex, it is noted that it is important that the extracts of these algae are used in combination with extracts of spirulina and *Crithmum maritimum,* which enhance the effect of red and brown algae extracts. *Crithmum maritimum* is not an algae, it is an umbelliferous plant, which grows on land, but in conditions similar to the habitats of algae. There is no biological antagonism between *Crithmum maritimum* and algae. Various amino acids can be used to produce the complex, both essential and nonessential ones. Amino acids are responsible for hair growth and hair resistance to external adverse factors. They make hair supple, shiny and attractive. Hair consists of 65% of proteins, which are based on amino acids. It is believed that it is amino acids that contribute to the penetration of beneficial substances into the hair and scalp. Moreover, they participate in the metabolism in the hair follicles and strengthen the hair roots, improve the water and protein balance of the skin, nourish and strengthen the hair. The amino acid component enhances and regenerates the structure of the hair, filling it with the necessary elements. Hydrolyzed wheat proteins moisturize, nourish and firm hair. Cysteine, which is part of proteins, provides a long conditioning effect, reduces hair fragility, soothes irritated skin and has an anti-inflammatory activity. The absorption of hydrolyzed wheat proteins on hair surface increases the hair plasticity, mainly due to their ability to retain fluid within the hair structure for a long time, and effectively restores split ends of hair and hair damaged by UV rays. All the components of the complex together can significantly increase the strength of the hair, and increase the density of hair growth. The disclosed protein-amino acid complex can be used in various hair care products, such as shampoos, balms, lotions, masks, etc.

The authors of the present invention confirmed that when using the complex according to RU2631620C1 all the claimed benefits are achieved. However, at the same time, there remains a need to provide alternative complexes based on other cosmetic substances that, on the one hand, would solve the above problems, and on the other, would make a significant contribution in this area, expanding the range of cosmetics. This is important because use of cosmetics by the consumer requires an individual approach. While some cosmetics are acceptable for some consumers, they may be unsuitable for some other consumers for various reasons, including allergies. Therefore, in order to satisfy the needs of the widest possible range of consumers, the search and creation of such alternative means is important.

The authors of the present invention conducted many studies to establish alternative combinations that would increase strength of the hair, as expected, by reducing porosity of the hair while increasing thickness of the hair, and provide an increase in the density of hair growth, their elasticity and gloss in order to expand the range of cosmetics and to offer the possibility to use them for a greater number of consumers.

### SUMMARY OF THE INVENTION

The invention is defined in the appended claims. In a first aspect, the claimed invention relates to a cosmetic composition for inclusion in a hair care product as defined in claim 1, said composition comprising a combination of the following components:
(a) a mineral complex containing a combination of cosmetically acceptable mineral salts, wherein said combination contains a magnesium salt and a salt selected from iron salt, calcium salt, copper salt, zinc salt or any combination thereof, wherein said magnesium salt is formed with a cosmetically acceptable monobasic or dibasic amino acid, and said iron salt, calcium salt, copper salt and/or zinc salt is formed with a cosmetically acceptable aldonic acid,
(b) an amino acid complex containing a combination of glycine and a cosmetically acceptable N-trialkyl derivative thereof, and
(c) an extraction complex containing a combination of water-glycerin extracts of *Chlorella* green algae and *Chondrus* red algae,
wherein components (a), (b) and (c) are in the ratio of (0.5-10):(10-60):(10-400).

In the claimed composition, the mineral complex may contain a cosmetic ingredient SEPITONIC M3.0^{™}.

In the claimed composition, the amino acid complex may contain a cosmetic ingredient PRODEW 500^{™}.

In the claimed composition, the amino acid complex may optionally contain a cosmetic ingredient GENENCARE OSMS BA^{™}.

The claimed composition may additionally contain a cosmetic ingredient HYDROTRITICUM WQ PE-LQ- (W_{D}) ^{™}.

In a second aspect, the claimed invention relates to a cosmetic product for hair care as defined in claim 2, characterized in that it comprises the cosmetic composition according to claim 1 and a cosmetically acceptable medium.

As defined in claims 3 and 4, said product according to the invention may be a shampoo for hair and scalp care or a balm for hair and scalp care.

The product according to the invention may be a hair and scalp care shampoo containing, % wt.:

| | |
|---|---|
| cosmetic ingredient | |
| SEPITONIC M3.0^{™} | from about 0.001 to about 0.5, |
| cosmetic ingredient | |
| GENENCARE OSMS BA^{™} | from about 0.02 to about 3.0, |
| cosmetic ingredient | |
| HYDROTRITICUM WQ PE-LQ-(WD) ^{™} | from about 0.02 to about 3.0, |
| cosmetic ingredient | |
| PRODEW 500^{™} | from about 0.01 to about 5.0. |

The shampoo according to the invention may contain these cosmetic ingredients and additional cosmetically acceptable ingredients and/or cosmetically acceptable medium.

The product according to the invention can be a hair and scalp care balm.

The product according to the invention can be a hair and scalp care balm containing, % wt.:

| | |
|---|---|
| cosmetic ingredient | |
| SEPITONIC M3.0^{™} | from about 0.001 to about 0.5, |
| cosmetic ingredient | |
| GENENCARE OSMS BA^{™} | from about 0.02 to about 3.0, |
| cosmetic ingredient | |
| HYDROTRITICUM WQ PE-LQ-(WD) ^{™} | from about 0.02 to about 3.0, |
| cosmetic ingredient | |
| PRODEW 500^{™} | from about 0.01 to about 5.0. |

The balm according to the invention may contain these cosmetic ingredients and additional cosmetically acceptable ingredients and/or cosmetically acceptable medium.

### SUMMARY OF THE FIGURES

Figure 1 illustrates the volume of hair before (A) and after (B) the use of shampoo with the composition according to the disclosure.
Figure 2 illustrates the structure of hair before (A) and after (B) the use of balm with the composition according to the disclosure.

### DETAILED DESCRIPTION OF THE INVENTION

It was unexpectedly found that the previously indicated advantages are possessed by a cosmetic composition intended for inclusion in a hair care product, comprising a combination of the following components:
(a) a mineral complex containing a combination of cosmetically acceptable mineral salts, wherein said combination contains a magnesium salt and a salt selected from iron salt, calcium salt, copper salt, zinc salt or any combination thereof, wherein said magnesium salt is formed with a cosmetically acceptable monobasic or dibasic amino acid, and said iron salt, calcium salt, copper salt and/or zinc salt is formed with a cosmetically acceptable aldonic acid,
(b) an amino acid complex containing a combination of glycine and a cosmetically acceptable N-trialkyl derivative thereof, and
(c) an extraction complex containing a combination of water-glycerin extracts of *Chlorella* green algae and *Chondrus* red algae,
wherein components (a), (b) and (c) are in the ratio of (0.5-10):(10-60):(10-400).

The positive effect on the hair according to the invention is due to the synergistic effect of biologically active substances.

It was found that effectiveness of the complex according to the invention depends on the presence of mineral components, namely, necessarily magnesium and at least one of iron, calcium, copper, zinc, or any combination thereof.

Although it is known that these minerals find use in the form of physiologically acceptable salts, it has been found that the best efficiency is achieved when a magnesium salt is formed with a cosmetically acceptable monobasic or dibasic amino acid, and an iron salt, calcium salt, copper salt and/or zinc salt is formed with a cosmetically acceptable aldonic acid. One skilled in the art will recognize which particular monobasic or dibasic amino acids are cosmetically acceptable. The acid may be, for example, aspartic acid. One skilled in the art will also understand which particular aldonic acids are cosmetically acceptable. The acid may be, for example, gluconic acid.

It was found that effectiveness of the complex according to the invention depends on the presence of amino acid components, namely, glycine and a cosmetically acceptable N-trialkyl derivative of glycine. One skilled in the art will understand which N-trialkyl derivatives of glycine are cosmetically acceptable. The derivative may be, for example, N,N,N-trimethylglycine.

It was found that effectiveness of the complex according to the invention depends on the presence of water-glycerin extracts of *Chlorella* green algae and *Chondrus* red algae. A person skilled in the art will understand that the content of active components (quantitative composition) in these extracts may vary depending on the extraction conditions, but it is also clear that the qualitative composition will be preserved. Moreover, the qualitative composition will be preserved when using various species of *Chlorella* green algae and *Chondrus* red algae. Such species may be *Chlorella vulgaris* and *Chondrus crispus.*

Thus, in one aspect, the present invention relates to a cosmetic composition (formulae) intended for inclusion in a hair care product. Such composition (formulae) contains or consists of several active ingredients, which can conditionally be attributed to mineral, amino acid and extraction complexes. The person skilled in the art understands that components in the composition are in a mixed form, and attribution of the component to a particular complex or "phase" is conditional and is given for the only purpose of better illustration.

As will be understood by a person skilled in the art, the cosmetic composition according to the disclosure is essentially an intermediate product intended to be included in a marketable product, a hair care product.

The cosmetic composition according to the invention contains or consists of a combination of the following components as defined in claim 1:
(a) a mineral complex containing a combination of cosmetically acceptable mineral salts, wherein said combination contains a magnesium salt and a salt selected from iron salt, calcium salt, copper salt, zinc salt or any combination thereof, wherein said magnesium salt is formed with a cosmetically acceptable monobasic or dibasic amino acid, and said iron salt, calcium salt, copper salt and/or zinc salt is formed with a cosmetically acceptable aldonic acid,
(b) an amino acid complex containing a combination of glycine and a cosmetically acceptable N-trialkyl derivative thereof, and
(c) an extraction complex containing a combination of water-glycerin extracts of *Chlorella* green algae and *Chondrus* red algae,
wherein components (a), (b) and (c) are in the ration of (0.5-10):(10-60):(10-400).

If the cosmetic composition according to the invention contains a combination of the above components, then it may also include cosmetically acceptable active or accessory substances.

As noted above, components (a), (b) and (c) are in the ratio of (0.5-10):(10-60):(10-400), which ensures the achievement of the claimed technical results.

In another aspect, the present invention relates to a cosmetic hair care product, which includes a cosmetic composition according to the invention and a cosmetically acceptable medium, as defined in claim 2. The product may be shampoo, balm, etc.

As an acceptable cosmetic medium, the product contains components selected from the group consisting of surfactants, solvents, thickeners, structure formers, moisturizers, preservatives, chelating agents, film formers, conditioning agents and functional additives, plant extracts and/or oils, and any combination thereof or other ingredients used in the manufacture of cosmetics. As an acceptable cosmetic medium, water, such as purified water, may be used.

When included in a cosmetic product for hair care the composition according to the invention will contain from about 0.001 to about 0.5 % wt. of component (a), from about 0.02 to about 5.0 % wt. of component (b), and from about 0.001 to about 20.0 % wt. of component (c).

A person skilled in the art will understand that the subject of the invention is not synthesis or other methods for producing components of the claimed composition and agent. All components are commercially available and can be purchased from suppliers of cosmetic ingredients both in Russia and abroad. For example, the magnesium salt may be an aspartic acid salt, the copper and zinc salts may be a gluconic acid salt and be part of the commercially available cosmetic ingredient SEPITONIC M3.0^{™}, SEPITONIC M3^{™}, SEPITONIC M4^{™} from SEPPIC^{™}. In this case, SEPITONIC M3.0^{™}, SEPITONIC M4^{™} from SEPPIC^{™} can be included in the composition of cosmetic products for hair care, such as shampoo or balm, in the amount from about 0.001 to about 0.5 % wt. For example, glycine may be part of the commercially available cosmetic ingredient Prodew 500^{™} from Ajinomoto^{™}. In this case, Prodew 500^{™} may be included in the composition of cosmetic products for hair care, such as shampoo or balm, in the amount from about 0.01 to about 5.0% wt. For example, the cosmetically acceptable N-trialkyl derivative of glycine may be betaine (N,N,N-trimethylglycine) and form part of the commercially available cosmetic ingredient Genencare OSMS BA^{™} from DuPont/Genencor^{™}. In this case, Genencare OSMS BA^{™} can be included in the composition of cosmetic products for hair care, such as shampoo or balm, in the amount from about 0.02 to about 5.0% wt. For example, water-glycerin extracts of *Chlorella* green algae and *Chondrus* red algae can be water-glycerin extracts of *Chlorella vulgaris* and *Chondrus crispus* and form part of a commercially available cosmetic ingredient. For example, the extract can be obtained and/or used as part of a commercially available product Water-glycerin extract Algae blend (Aqua, Glycerin, *Laminaria Saccharina* Extract, *Cystoseira Compressa* Extract, *Fucus Vesiculosus* Extract, *Chlorella vulgaris* Extract, *Chondrus crispus* Extract). In this case, the Water-glycerin extract Algae blend can be included in the composition of cosmetic products for hair care, such as shampoo or balm, in the amount from about 0.05 to about 20.0 % wt.

The well-known commercially available cosmetic ingredient is a three-action chrono-activating and anti-radical agent for skin and hair care SEPITONIC M3.0^{™} with the following properties: chrono-activation due to increased cellular respiration, ATP production and epidermal renewal within 24 hours, derma protection (fibroblasts and extracellular matrix) from the glycation process, protection against oxidative stress, free radical blocking and activation of intercellular communication. The mineral complex is Magnesium Aspartate, Zinc Gluconate, Copper Gluconate (INCI name: Magnesium Aspartate & Zinc Gluconate & Copper Gluconate) and can be used as part of hair care products.

A well-known commercially available cosmetic ingredient is highly purified anhydrous betaine (INCI name: Betaine) GENENCARE OSMS BA^{™} derived from sugar beets. The trimethylglycine-based agent may find application in hair care products.

A well-known commercially available cosmetic ingredient is Hydroxypropyltrimonium Hydrolyzed Wheat Protein (CAS: 145269-34-7, INCI name: Aqua and Hydroxypropyltrimonium Hydrolyzed Wheat Protein) HYDROTRITICUM WQ PE-LQ-(WD) ^{™} The conditioning agent may find application in the composition of hair care products.

A well-known commercially available cosmetic ingredient PRODEW 500^{™} is a mixture of sodium L-pyrrolidone carboxylate (PCA), sodium lactate, L-arginine, L-aspartic acid, L-pyrrolidone carboxylate (PCA), glycine, L-alanine, L-serine, L-valine, L-proline, L-threonine, L -isoleucine, L-histidine, L-phenylalanine and water (INCI Name: Sodium L-Pyrrolidonecarboxilate (PCA) & Sodium Lactate & L-Arginine & L-Aspartic Acid & L-Pyrrolidonecarboxilic Acid (PCA) & Glycine & L-Alanine & L-Serine & L-Valine & L-Proline & L-Threonine & L-Isoleucine & L-Histidine & L-Phenylalanine & Aqua). The agent may find application in hair care products.

A well-known commercially available cosmetic ingredient is a water-glycerin extract of kelp, cystozira, fucus, chlorella, and Irish moss blend (INCI name: Laminaria Saccharina Extract & Cystoseira Compressa Extract & Fucus Vesiculosus Extract & Chlorella vulgaris Extract & Chondrus crispus Extract & Euxyl K 712 1% & Glycerin & Aqua). The positive effect on the hair according to the disclosure is due to the synergistic effect of the biologically active substances.

As will be understood by a person skilled in the art, the cosmetic composition according to the invention is essentially an intermediate product intended to be included in a marketable product, a hair care product.

The shampoo for hair and scalp care according to the invention contains, % wt.: cosmetic ingredient SEPITONIC M3.0^{™} from about 0.001 to about 0.5 (such amount as from about 0.005 to about 0.45, from about 0.01 to about 0.4, from about 0.015 to about 0.35, from about 0.02 to about 0.3, from about 0.025 to about 0.25, from about 0.03 to about 0.2, from about 0.035 to about 0.15, from about 0.04 to about 0.1, from about 0.045 to about 0.05, for example, about 0.001, 0.003, 0.005, 0.007, 0.009, 0.01, 0.03, 0.05, 0, 07, 0.09, 0.1, 0.2, 0.3, 0.4, or about 0.5), cosmetic ingredient GENENCARE OSMS BA^{™} from about 0.02 to about 3.0 (such amount as about 0.025 to about 3.0, from about 0.03 to about 2.5, from about 0.035 to about 2.0, from about 0.04 to about 1.5, from about 0.045 to about 1.0, from about 0.05 to about 0.5, from about 0.055 to about 0.1, from about 0.06 to about 0.08, for example, about 0.02, 0.05, 0.08, 0.1, 0.3, 0.5, 0, 7, 0.9, 1.0, 1.2, 1.4, 1.6, 1.8, 2.0, 2.2, 2.4, 2.6, 2.8, or about 3.0), cosmetic ingredient HYDROTRITICUM WQ PE-LQ- (WD) ^{™} from about 0.02 to about 3.0 (such amount as from about 0.025 to about 3.0, from about 0.03 to about 2.5, from about 0.035 to about 2.0, from about 0.04 to about 1.5, from about 0.045 to about 1.0, from about 0.05 to about 0.5, from about 0.055 to about 0.1, from about 0 06 to about 0.08, for example, about 0.02, 0.05, 0.08, 0.1, 0.3, 0.5, 0.7, 0.9, 1.0, 1.2, 1.4, 1.6, 1.8, 2.0, 2.2, 2.4, 2.6, 2, 8 or about 3.0), cosmetic ingredient PRODEW 500^{™} from about 0.01 to about 5.0 (such amount as from about 0.01 to about 4.5, from about 0.02 to about 4.0, from about 0.03 to about 3.5, from about 0.04 to about 3.0, from about 0.05 to about 2.5, from about 0.06 to about 2.0, from about 0.07 to about 1.5, from about 0.08 to about 1.0, from about 0.09 to about 0.9, from about 0.1 to about 0.8, from about 0.3 to about 0.6, for example, about 0.01, 0.02, 0.05, 0.08, 0.1, 0.3, 0.5, 0.7, 0.9, 1.0, 1.2, 1.4, 1 6, 1.8, 2.0, 2.2, 2.4, 2.6, 2.8, 3.0, 3.2, 3.4, 3.6, 3.8, 4.0 , 4.2, 4.4, 4.6, 4.8 or about 5.0)

In an embodiment of the invention, the shampoo may contain the said cosmetic ingredients and additional cosmetically acceptable ingredients and/or a cosmetically acceptable medium.

The balm for hair and scalp care according to the disclosure contains, % wt.: cosmetic ingredient SEPITONIC M3.0^{™} from about 0.001 to about 0.5 (kelp, cystozira, fucus, chlorella, Irish moss from about 0.005 to about 0.45, from about 0.01 to about 0.4, from about 0.015 to about 0.35, from about 0.02 to about 0.3, from about 0.025 to about 0.25, from about 0.03 to about 0.2, from about 0.035 to about 0.15, from about 0.04 to about 0.1, from about 0.045 to about 0.05, for example, about 0.001, 0.003, 0.005, 0.007, 0.009, 0.01, 0.03, 0.05, 0, 07, 0.09, 0.1, 0.2, 0.3, 0.4, or about 0.5), cosmetic ingredient GENENCARE OSMS BA^{™} from about 0.02 to about 3.0 (such amount as about 0.025 to about 3.0, from about 0.03 to about 2.5, from about 0.035 to about 2.0, from about 0.04 to about 1.5, from about 0.045 to about 1.0, from about 0.05 to about 0.5, from about 0.055 to about 0.1, from about 0.06 to about 0.08, for example, about 0.02, 0.05, 0.08, 0.1, 0.3, 0.5, 0, 7, 0.9, 1.0, 1.2, 1.4, 1.6, 1.8, 2.0, 2.2, 2.4, 2.6, 2.8, or about 3.0), cosmetic ingredient HYDROTRITICUM WQ PE-LQ-(WD) ^{™} from about 0.02 to about 3.0 (such amount as from about 0.025 to about 3.0, from about 0.03 to about 2.5, from about 0.035 to about 2.0, from about 0.04 to about 1.5, from about 0.045 to about 1.0, from about 0.05 to about 0.5, from about 0.055 to about 0.1, from about 0 .06 to about 0.08, for example, about 0.02, 0.05, 0.08, 0.1, 0.3, 0 5, 0.7, 0.9, 1.0, 1.2, 1.4, 1.6, 1.8, 2.0, 2.2, 2.4, 2.6, 2.8 or about 3.0), cosmetic ingredient PRODEW 500^{™} from about 0.01 to about 5.0 (such amount as from about 0.01 to about 4.5, from about 0.02 to about 4.0, from about 0.03 to about 3.5, from about 0.04 to about 3.0, from about 0.05 to about 2.5, from about 0.06 to about 2.0, from about 0.07 to about 1 5, from about 0.08 to about 1.0, from about 0.09 to about 0.9, from about 0.1 to about 0.8, from about 0.3 to about 0.6, for example, about 0.01, 0.02, 0.05, 0.08, 0.1, 0.3, 0.5, 0.7, 0.9, 1.0, 1.2, 1.4, 1, 6, 1.8, 2.0, 2.2, 2.4, 2.6, 2.8, 3.0, 3.2, 3.4, 3.6, 3.8, 4.0, 4.2, 4.4, 4.6, 4.8 or about 5.0).

In an embodiment of the invention, the balm may contain the specified cosmetic ingredients and additional cosmetic acceptable ingredients and/or cosmetically acceptable medium. Such additional cosmetic acceptable ingredients and/or cosmetically acceptable medium may include ARLASILK PTM-LQ-(AP) ^{™}, Celquat SC-240C Polyquartenium 10^{™}, IT ROSE Fragrance^{™}, Lamesoft PO 65^{™} Surfactant Mixture, Mel Extract Gly^{™}, MG 60^{™} (maltooligosyl glucoside + hydrogenated starch hydrolyzate), Keramare 733000^{™} Active Complex, VENUCEANE^{™} Active Complex, Vitamin D-Panthenol D-Panthenol 75W BASF^{™}, Purified Water, Aloe Vera Gel 10x1 Gel, Glycerin (99.5%; 99.7%), Cocamidopropylate 40-45%) Surfactant, Elfan AT 84G^{™} Sodium Cocoylisothionate Surfactant, Optiphen BD^{™} Preservative, Plantacare 1200 UP^{™} Surfactant Lauryl Glucoside, Citric Acid, Sodium Lauroyl Methylisothionate Iselux^{™} Surfactant, Jaguar C 162^{™} Guar Gum Derivative, Tetrasodium Glutamate Diacetate Dissolvine GL-47-S^{™} Complexing Agent, Orange Oil Soain Ventos^{™} Essential Oil, Basil Essential Oil 9801 2946^{™}, Lavender Essential Oil 200114^{™}. Alternatively, such additional cosmetically acceptable ingredients and/or cosmetically acceptable medium may include IT ROSE^{™} Fragrance, Invita^{™} Refined Macadamia Nut Oil, Vitamin D-Panthenol 75W BASF^{™}, Purified Water, Natrosol 250 HHR^{™} Hydroxyethyl Cellulose, Fatty Alcohols C16-C18 (Ginol 1618 TA^{™}), Begentrimonium Chloride Genamin KDMP^{™} Cationic Surfactant, Cetrimonium Chloride 30% Arquad^{™} 16-29 Cation Surfactant, Crodazoquat MCC^{™} Conditioner, Optiphen BD^{™} Preservative, Lutein 5% Microencapsulated Dye, Refined Shea Butter 000-888, Lactic Acid 80%, Natural Fragrance with Cinnamon Essential Oil, Bergenia Root Dry Extract, Dissolvine GL-47-S^{™} Tetrasodium Glutamate Diacetate Complexing Agent, *Caulepra Lentillifera* Green Algae Extract, Loquat Leaf Extract Powder 130926 (SF-LLE001^{™}), Emolent Octyl Dodecanol Eutanol G, ^{™} and Orange Oil Spain Ventos^{™} Essential Oil.

When the cosmetic composition according to the invention contains a combination of the above components, it may also include cosmetically acceptable active or accessory substances. As an acceptable cosmetic medium, it contains components selected from the group consisting of surfactants, solvents, thickeners, moisturizers, preservatives, chelating agents, film formers, conditioning additives and functional additives, herbal extracts and/or oils, and any combination thereof. As an acceptable cosmetic medium, water, such as purified water, may be used.

A person skilled in the art will understand that the subject of the invention is not synthesis or other methods for manufacturing components of the claimed composition and product. All components are commercially available and can be purchased from suppliers of cosmetic ingredients both in Russia and abroad. The active ingredient may be included in the component, either on its own or in the composition. The ratio of the ingredients in the component composition for inclusion into the shampoo or balm according to this invention is definite and established by manufacturer of the component. SEPITONIC M3.0^{™} as a commercially available component can be included in the composition of cosmetic products for hair care, such as shampoo or balm, in the amount from about 0.001 to about 0.5 % wt. PRODEW 500^{™} as a commercially available component can be included in the composition of cosmetic products for hair care, such as shampoo or balm, in the amount from about 0.01 to about 5.0 % wt. GENENCARE OSMS BA^{™} as a commercially available component can be included in the composition of cosmetic products for hair care, such as shampoo or balm, in the amount from about 0.02 to about 3.0 % wt. Water-glycerin extract Algae blend (Aqua, Glycerin, *Laminaria Saccharina* Extract, *Cystoseira Compressa* Extract, *Fucus Vesiculosus* Extract, *Chlorella vulgaris* Extract, *Chondrus crispus* Extract) manufactured by Vtoroy Tsekh LLC (Russia) as a commercially available component can be included in the cosmetic products for hair care, such as shampoo or balm, in the amount from about 0.05 to about 20.0 % wt. HYDROTRITICUM WQ PE-LQ- (WD)^{™} as a commercially available component can be included in the composition of cosmetic products for hair care, such as shampoo or balm, in the amount from about 0.02 to about 3.0 wt.%.

In a first aspect, the claimed invention relates to a cosmetic composition for inclusion in a hair care product as defined in claim 1, said composition comprising a combination of the following components:
(a) a mineral complex containing a combination of cosmetically acceptable mineral salts, wherein said combination contains a magnesium salt and a salt selected from iron salt, calcium salt, copper salt, zinc salt or any combination thereof, wherein said magnesium salt is formed with a cosmetically acceptable monobasic or dibasic amino acid, and said iron salt, calcium salt, copper salt and/or zinc salt is formed with a cosmetically acceptable aldonic acid,
(b) an amino acid complex containing a combination of glycine and a cosmetically acceptable N-trialkyl derivative thereof, and
(c) an extraction complex containing a combination of water-glycerin extracts of *Chlorella* green algae and *Chondrus* red algae,
wherein components (a), (b) and (c) are in the ratio of (0.5-10):(10-60):(10-400).

In the claimed composition, the mineral complex may contain a cosmetic ingredient SEPITONIC M3.0^{™}.

In the claimed composition, the amino acid complex may contain a cosmetic ingredient PRODEW 500^{™}.

In the claimed composition, the amino acid complex may optionally contain a cosmetic ingredient GENENCARE OSMS BA^{™}.

The claimed composition may additionally contain a cosmetic ingredient HYDROTRITICUM WQ PE-LQ- (WD)^{™}.

In a second aspect, the claimed invention relates to a cosmetic product for hair care as defined in claim 2, characterized in that it comprises the cosmetic composition according to the claim 1, and a cosmetically acceptable medium.

As defined in claims 3 and 4, said product may be shampoo for hair and scalp care.

The product according to the invention may be a hair and scalp care shampoo containing, % wt.:

| | |
|---|---|
| cosmetic ingredient | |
| SEPITONIC M3.0^{™} | from about 0.001 to about 0.5, |
| cosmetic ingredient | |
| GENENCARE OSMS BA^{™} | from about 0.02 to about 3.0, |
| cosmetic ingredient | |
| HYDROTRITICUM WQ PE-LQ-(WD) ^{™} | from about 0.02 to about 3.0, |
| cosmetic ingredient | |
| PRODEW 500^{™} | from about 0.01 to about 5.0. |

The shampoo according to the invention may contain these cosmetic ingredients and additional cosmetically acceptable ingredients and/or cosmetically acceptable medium.

The product according to the invention can be a hair and scalp care balm.

The product according to the invention can be a hair and scalp care balm containing, % wt.:

| | |
|---|---|
| cosmetic ingredient | |
| SEPITONIC M3.0^{™} | from about 0.001 to about 0.5, |
| cosmetic ingredient | |
| GENENCARE OSMS BA^{™} | from about 0.02 to about 3.0, |
| cosmetic ingredient | |
| HYDROTRITICUM WQ PE-LQ-(WD) ^{™} | from about 0.02 to about 3.0, |
| cosmetic ingredient | |
| PRODEW 500^{™} | from about 0.01 to about 5.0. |

The balm according to the invention may contain these cosmetic ingredients and additional cosmetically acceptable ingredients and/or cosmetically acceptable medium.

### EXAMPLES

### Example 1

Illustrative formulations of shampoos containing the first aspect of the claimed invention relating to a cosmetic composition for inclusion in a hair care product are given in Tables 1-4.

**Table 1. Formulation #1sh.**

| **Ingredient** | **%wt.** |
|---|---|
| Cosmetic composition according to the first aspect | up to 1.80000 |
| Anionic surfactants | up to 10.0000 |
| Perfume composition (synthetic, natural or a mixture thereof) | up to 0.5000 |
| Nonionic surfactants | up to 10.000 |
| Active additives (D-panthenol, honey extract, thermophilic enzymes) | up to 10.000 |
| Amphoteric surfactants | up to 6.0000 |
| Preservative | up to 1.000 |
| Accessory substances or cosmetically acceptable medium | the rest |

**Table 2. Formulation #2sh.**

| **Ingredient** | **%wt.** |
|---|---|
| Cosmetic composition according to the first aspect | up to 1.80000 |
| Anionic surfactants | up to 10.0000 |
| Perfume composition (synthetic, natural or a mixture thereof) | up to 0.5000 |
| Nonionic surfactants | up to 10.000 |
| Active additives (D-panthenol, honey extract, green algae extract) | up to 10.000 |
| Amphoteric surfactants | up to 6.0000 |
| Preservative | up to 1.000 |
| Accessory substances or cosmetically acceptable medium | the rest |

**Table 3. Formulation #3sh.**

| **Ingredient** | **%wt.** |
|---|---|
| Cosmetic composition according to the first aspect | up to 1.80000 |
| Anionic surfactants | up to 10.0000 |
| Perfume composition (synthetic, natural or a mixture thereof) | up to 0.5000 |
| Nonionic surfactants | up to 10.000 |
| Active additives (D-panthenol, honey extract, wild sugar beet extract) | up to 10.000 |
| Amphoteric surfactants | up to 6.0000 |
| Preservative | up to 1.000 |
| Accessory substances or cosmetically acceptable medium | the rest |

**Table 4. Formulation #4sh.**

| **Ingredient** | **%wt.** |
|---|---|
| Cosmetic composition according to the first aspect | up to 1.80000 |
| Anionic surfactants | up to 10.0000 |
| Perfume composition (synthetic, natural or a mixture thereof) | up to 0.5000 |
| Nonionic surfactants | up to 10.000 |
| Active additives (D-panthenol, honey extract, *Limonium Narbonense* extract) | up to 10.000 |
| Amphoteric surfactants | up to 6.0000 |
| Preservative | up to 1.000 |
| Accessory substances or cosmetically acceptable medium | the rest |

### Example 2

Illustrative formulations of balsams containing the first aspect of the claimed invention relating to a cosmetic composition intended for inclusion in a hair care product are given in Tables 5-8.

**Table 5. Formulation #1b.**

| **Ingredient** | **%wt.** |
|---|---|
| Cosmetic composition according to the first aspect | up to 1.80000 |
| Emulsifiers | up to 10.0000 |
| Perfume composition (synthetic, natural or a mixture thereof) | up to 0.5000 |
| Emollients | up to 10.000 |
| Active additives (D-panthenol, honey extract, thermophilic enzymes) | up to 10.000 |
| Oils | up to 6.0000 |
| Preservative | up to 1.000 |
| Texturizers | up to 10.000 |
| Accessory substances or cosmetically acceptable medium | the rest |

**Table 6. Formulation #2b.**

| **Ingredient** | **%wt.** |
|---|---|
| Cosmetic composition according to the first aspect | up to 1.80000 |
| Emulsifiers | up to 10.0000 |
| Perfume composition (synthetic, natural or a mixture thereof) | up to 0.5000 |
| Emollients | up to 10.000 |
| Active additives (D-panthenol, honey extract, green algae extract) | up to 10.000 |
| Oils | up to 6.0000 |
| Preservative | up to 1.000 |
| Texturizers | up to 10.000 |
| Accessory substances or cosmetically acceptable medium | the rest |

**Table 7. Formulation #3b.**

| **Ingredient** | **%wt.** |
|---|---|
| Cosmetic composition according to the first aspect | up to 1.80000 |
| Emulsifiers | up to 10.0000 |
| Perfume composition (synthetic, natural or a mixture thereof) | up to 0.5000 |
| Emollients | up to 10.000 |
| Active additives (D-panthenol, honey extract, *Limonium Narbonense* extract) | up to 10.000 |
| Oils | up to 6.0000 |
| Preservative | up to 1.000 |
| Texturizers | up to 10.000 |
| Accessory substances or cosmetically acceptable medium | the rest |

**Table 8. Formulation #4b.**

| **Ingredient** | **%wt.** |
|---|---|
| Cosmetic composition according to the first aspect | up to 1.80000 |
| Emulsifiers | up to 10.0000 |
| Perfume composition (synthetic, natural or a mixture thereof) | up to 0.5000 |
| Emollients | up to 10.000 |
| Active additives (D-panthenol, honey extract, *Limonium Narbonense* extract) | up to 10.000 |
| Oils | up to 6.0000 |
| Preservative | up to 1.000 |
| Texturizers | up to 10.000 |
| Accessory substances or cosmetically acceptable medium | the rest |

Example 3. Method No. 1 for the preparation of the first aspect of the claimed invention relating to a cosmetic composition intended for inclusion in a hair care product.

The mineral complex was prepared by mixing of the required amounts of magnesium salt with cosmetically acceptable monobasic or dibasic amino acid and one or more of iron salt, calcium salt, copper salt and/or zinc salt with cosmetically acceptable aldonic acid.

The amino acid complex was prepared by mixing of the required amounts of glycine and cosmetically acceptable N-trialkyl derivative thereof.

The extraction complex was prepared by mixing of the required amounts of water-glycerin extracts of *Chlorella* green algae and *Chondrus* red algae.

The obtained mineral, amino acid and extraction complexes were combined in the required proportions and mixed to obtain the composition according to the invention.

Example 3. Method No. 2 for the preparation of the first aspect of the claimed invention relating to a cosmetic composition intended for inclusion in a hair care product.

The composition of the invention was prepared by mixing of the required amounts of magnesium salt with cosmetically acceptable monobasic or dibasic amino acid and one or more of iron salt, calcium salt, copper salt and/or zinc salt with cosmetically acceptable aldonic acid, glycine and cosmetically acceptable N-trialkyl derivative thereof, water-glycerin extracts of *Chlorella* green algae and *Chondrus* red algae, and the components were combined in the required proportions.

Example 4. The method for preparation of the second aspect of the claimed invention relating to a product for hair care.

A hair care product, such as, for example, shampoo or balm, was prepared by mixing of the required amounts of the composition according to the invention and other cosmetically acceptable components, such as, for example, the components shown in Tables 1-8.

Methodology for studying the effects of use of the hair care products according to the invention.

Shampoos #1-4 and Shampoo #0 (control: Shampoo #1 without adding the composition according to the invention) were examined in a test center.

Groups of 15 subjects were selected for testing each shampoo.

At the beginning of the study, each volunteer was given a set of one bottle of shampoo.

The volunteers used the products at home in accordance with the application method recommended by the manufacturer. The frequency of use was typical for each volunteer in the usual regimen of care for the scalp. The total duration of use of the tested products was 60 calendar days (2 months).

The test results were recorded at the beginning and at the end of the study and the % change was calculated in accordance with the same methodology as in RU2631620C1 (see Table 9).
- hair strength (up to + 10% depending on shampoo #);
- hair shaft thickness (up to + 6.5% depending on shampoo #);
- gloss of hair (up to + 6% depending on shampoo #);
- hair growth density (up to + 6.8% depending on shampoo #).

**Table 9.**

| **Shampoo #** | **0** | **1** | **2** | **3** | **4** |
|---|---|---|---|---|---|
| Strength (resistibility), g/hair | | | | | |
| changes, % | +1.5 | +6.3 | +7.5 | +10.0 | +8.9 |
| changes relative to #0 | | 4.2 times | 5 times | 7.6 times | 6.8 times |
| Hair porosity | | | | | |
| changes, % | almost normal | normalized | normalized | normalized | normalized |
| Hair growth density, hair/cm² | | | | | |
| changes, % | +1.5 | +5.2 | +4.7 | +6.3 | +6.8 |
| changes relative to #0 | | 3.4 times | 3.1 times | 4.2 times | 4.5 times |
| Hair shaft thickness | | | | | |
| changes, % | +1.3 | +5.2 | +5.4 | +6.5 | +6.2 |
| changes relative to #0 | | 3.9 times | 4.1 times | 5 times | 4.7 times |
| Elasticity | | | | | |
| changes, % | +0.4 | +0.7 | +0.7 | +0.8 | +0.8 |
| changes relative to #0 | | 1.7 times | 1.7 times | 2 times | 2 times |
| Gloss | | | | | |
| changes, % | +3.0 | +5.5 | +5.0 | +5.0 | +6.0 |
| changes relative to #0 | | 1.8 times | 1.6 times | 1.6 times | 2 times |

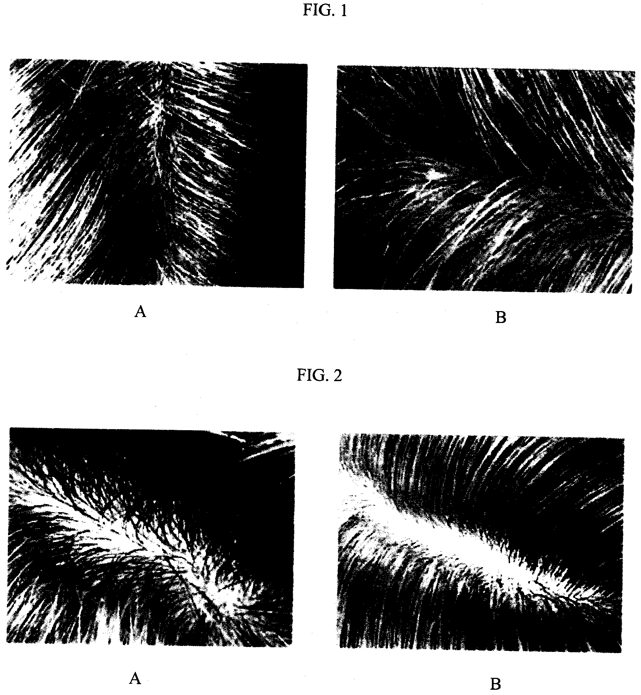

As follows from the above data, the tests confirmed an increase in hair strength, a decrease in hair porosity and an increase in the thickness of an individual hair, as well as an increase in elasticity and gloss. The volume of hair before (A) and after (B) using the shampoo with the claimed complex according to the invention is illustrated in Fig. 1.

To conduct a balm efficacy study, volunteers were given Balm # 1-4 and Balm # 0 kits (control: Balm # 0 without adding the composition of the invention) were examined in a test center. Sample # 0 was used as a shampoo to prepare hair for the study of balsams. For the study were selected groups of subjects of 15 people for each set.

Volunteers used the products at home in accordance with the application method as recommended by the manufacturer. The frequency of use was typical for each volunteer in the usual regimen of care for the scalp. The total duration of use of the tested funds was 60 calendar days (2 months).

The test results are recorded in Table 10.
- hair strength (up to +20% depending on balm #);
- hair shaft thickness (up to +11% depending on balm #);
- gloss of hair (up to +21% depending on balm #);
- hair growth density (up to +12% depending on balm #).

**Table 10.**

| **Balm #** | **0** | **1** | **2** | **3** | **4** |
|---|---|---|---|---|---|
| Strength (resistibility), g/hair | | | | | |
| changes, % | +1.5 | +15.0 | +16.0 | +20.0 | +18.0 |
| changes relative to #0 | | 10 times | 10.6 times | 13.3 times | 12 times |
| Hair porosity | | | | | |
| changes, % | almost normal | normalized | normalized | normalized | normalized |
| Hair growth density, hair/cm² | | | | | |
| changes, % | +1.5 | +10.0 | +9.0 | +11.0 | +12.0 |
| changes relative to #0 | | 6.6 times | 6 times | 7.3 times | 8 times |
| Hair shaft thickness | | | | | |
| changes, % | +1.8 | +10.0 | +10.0 | +11.0 | +11.0 |
| changes relative to #0 | | 5.5 times | 5.5 times | 6.1 times | 6.1 times |
| Elasticity | | | | | |
| changes, % | +0.5 | +1.0 | +1.0 | +1.1 | +1.0 |
| changes relative to #0 | | 2 times | 2 times | 2.2 times | 2 times |
| Gloss | | | | | |
| changes, % | +3.5 | +17.0 | +21.0 | +17.0 | +18.0 |
| changes relative to #0 | | 4.8 times | 6 times | 4.8 times | 5.1 times |

As follows from the above data, the tests confirmed an increase in hair strength, a decrease in hair porosity and an increase in the thickness of an individual hair, as well as an increase in elasticity and gloss. A comparison of strength of the hair before (A) and after (B) use of the balm with the complex according to the invention is illustrated in Fig. 2.

### Example 5

The shampoo formulation of the product according to the invention, which is a shampoo for hair and scalp care containing the cosmetic ingredient SEPITONIC M3.0^{™}, the cosmetic ingredient GENENCARE OSMS BA^{™}, the cosmetic ingredient HYDROTRITICUM WQ PE-LQ-(WD) ^{™} and the cosmetic ingredient PRODEW 500^{™}, is illustrated in Table 11.

**Table 11.**

| **Ingredient** | **%wt.** |
|---|---|
| Cosmetic composition according to the disclosure | 1.75 |
| Accessory substances or cosmetically acceptable medium | the rest |

The shampoo formulation of the product according to the invention, which is a hair and scalp care balm containing the cosmetic ingredient SEPITONIC M3.0^{™}, the cosmetic ingredient GENENCARE OSMS BA^{™} the cosmetic ingredient HYDROTRITICUM WQ PE-LQ-(WD) ^{™} and the cosmetic ingredient PRODEW 500^{™}, is illustrated in Table 12.

**Table 12.**

| **Ingredient** | **%wt.** |
|---|---|
| Cosmetic composition according to the disclosure | 1.20 |
| Accessory substances or cosmetically acceptable medium | the rest |

### Example 6

Where the product according to the invention contains the cosmetic ingredient SEPITONIC M3.0^{™}, the cosmetic ingredient GENENCARE OSMS BA^{™}, and the cosmetic ingredient HYDROTRITICUM WQ PE-LQ-(WD) ^{™}, the methodology for studying the effects of using the agent is illustrated by the following example.

Shampoo # 0 (control: Shampoo # 1 without the composition according to the invention) and Shampoo # 1 were examined in a test center. Groups of subjects, 60 people each, were selected for testing each shampoo. At the beginning of the study, each subject was given one bottle of shampoo. The subjects used the products at home in accordance with the application method recommended by the manufacturer. Frequency of use was typical for each subject in the usual regimen of caring for the scalp. The total duration of use of the tested products was 30 calendar days (1 month). The test results were recorded at the beginning and at the end of the study, the % change was calculated and summarized in Table 13.

**Table 13.**

| **Shampoo #** | **0** | **1** |
|---|---|---|
| Strength (resistibility), g/hair | | |
| changes, % | +1.1 | +12.0 |
| Hair porosity | | |
| changes, % | almost normal | normalized |
| Hair growth density, hair/cm² | | |
| changes, % | +1.2 | +8.0 |
| Hair shaft thickness | | |
| changes, % | +1.3 | +8.0 |
| Elasticity | | |
| changes, % | +0.4 | +1.0 |
| Gloss | | |
| changes, % | +2.8 | +9.0 |

As follows from the above data, the tests confirmed an increase in hair strength, a decrease in hair porosity and an increase in thickness of an individual hair, as well as an increase in elasticity and gloss.

### References

1. Colavincenzo ML. Practice and Educational Gaps in Dermatology: Disorders of the Hair // Dermatol Clin., 2016, 34, 275-279
2. Chiu CH, Huang SH, Wang HM. A Review: Hair Health, Concerns of Shampoo Ingredients and Scalp Nourishing Treatments // Curr Pharm Biotechnol., 2015, 16, 1045-1052
3. Sinclair R.D. Healthy hair: What is it? // Journal of investigative Dermatology Symposium Proceedings, 2007, 12, 2-5
4. (a) Wall R.A., Hunter L.D. Normal adult hair - structure and properties // Cosmet. Perf., 1974, 89, 31-36 (b) R. de Crassia Comis Wagner, I. Joekes. Hair protein removal by sodium dodecyl sulfate // Colloids and Surfaces B: Biointerfaces, 2005, 41, 7-14
5. C.R. Robbins (Ed.), Chemical and Physical Behavior of Human Hair, Springer-Verlag, New York, 1991 (Chapters 1, 2 and 5)
6. Feughelman M. Intermicrofibrillar linkages in α-keratin fibers // Text Res J., 1979, 49, 704-709
7. Wagner R.C., Joekes I. Hair protein removal by sodium dodecyl sulfate. Colloids and Surfaces // Biointerfaces, 2005, 41, 7-14
8. Jones L.N., Simon M., Watts N.R., Booy F.P., Steven A.C., Parry D.A.D. Intermediate filament structure: hard α-keratin // Biophys. Chem., 1997, 68, 83-93
9. Naito S., Arai K., Hirano M., Nagasawa N., Sakamoto M. Crosslinking structure of keratin. V. Number and type of crosslinks in microstructures of untreated and potassium cyanide treated human hair// J. Appl. Polym. Sci., 1996, 61, 1913-1925
10. Naito S., Arai K. Type and location of SS linkages in human hair and their relation to fiber properties in water // J. Appl. Polym. Sci., 1996, 61, 2113-2118
11. Bhushan B, Chen N. AFM studies of environmental effects on nanomechanical properties and cellular structure of human hair // Ultramicroscopy, 2006, 106, 755-764
12. J.R. Smith, J.A. Swift. Lamellar subcomponents of the cuticular cell membrane complex of mammalian keratin fibres show friction and hardness contrast by AFM // J. Microsc., 2002, 206, 182-193
13. L.N. Jones, D.E. Rivetti.The role of 18-methylicosanoic acid in the structure and formation of mammalian hair fibers //Micron.,1997, 28,469-485
14. K. Roper, J. Fohles, D. Peters. Morphological Composition of the Cuticle from Chemically Treated Wool // Text. Res. J, 1984, 54 139-143
15. E.G. Bendit, M. Feughelman (Eds.), Keratin, Encyclopedia of Polymer Science and Technology, 1967, vol. 8, (Chapter 1)
16. Hashimoto K. The structure of human hair // Clinics in Dermatology, 1988, 6, 4, 7-21 Marsh J.M., Brown M., Felts T., Hutton H., Vatter M., Whitaker S., Wireko F., Styczynski P., Li C., Henry I. Gel Network Shampoo Formulation and Hair Health Benefits // International Journal of Cosmetic Science, DOI:10.1111/ics.12409

## Claims

1. A cosmetic composition for inclusion in a hair care product, comprising a combination of the following components:
(a) a mineral complex containing a combination of cosmetically acceptable mineral salts, wherein said combination contains a magnesium salt and a salt selected from iron salt, calcium salt, copper salt, zinc salt or any combination thereof, wherein said magnesium salt is formed with a cosmetically acceptable monobasic or dibasic amino acid, and said iron salt, calcium salt, copper salt and/or zinc salt is formed with a cosmetically acceptable aldonic acid,
(b) an amino acid complex containing a combination of glycine and a cosmetically acceptable N-trialkyl derivative thereof, and
(c) an extraction complex containing a combination of water-glycerin extracts of *Chlorella* green algae and *Chondrus* red algae,
wherein components (a), (b) and (c) are in the ratio of (0.5-10):(10-60):(10-400).

2. A cosmetic product for hair care, which includes a cosmetic composition in accordance with claim 1 and a cosmetically acceptable medium.

3. The product according to claim 2, which is a shampoo for hair and scalp care.

4. The product according to claim 2, which is a balm for hair and scalp care.

## Patentansprüche

1. Kosmetische Zusammensetzung zur Zugabe zu einem Haarpflegeprodukt, umfassend eine Kombination der folgenden Bestandteile:
(a) einen mineralischen Komplex, der eine Kombination aus kosmetisch verträglichen Mineralsalzen enthält, wobei die Kombination ein Magnesiumsalz und ein Salz enthält, das aus Eisensalz, Calciumsalz, Kupfersalz, Zinksalz oder einer beliebigen Kombination davon ausgewählt ist, wobei das Magnesiumsalz mit einer kosmetisch verträglichen monobasischen oder dibasischen Aminosäure gebildet ist und das Eisensalz, Calciumsalz, Kupfersalz und/oder Zinksalz mit einer kosmetisch verträglichen Aldonsäure gebildet ist,
(b) einen Aminosäurekomplex, der eine Kombination aus Glycin und einem kosmetisch verträglichen N-Trialkylderivat davon enthält, und
(c) einen Extraktionskomplex, der eine Kombination aus Wasser-Glycerin-Extrakten aus *Chlorella-Grünalgen* und *Chondrus-Rotalgen* enthält,
wobei die Komponenten (a), (b) und (c) in einem Verhältnis von (0,5-10) : (10-60) : (10-400) vorliegen.

2. Kosmetisches Produkt zur Haarpflege, das eine kosmetische Zusammensetzung nach Anspruch 1 und ein kosmetisch akzeptables Medium enthält.

3. Produkt nach Anspruch 2, das ein Shampoo zur Haar- und Kopfhautpflege ist.

4. Produkt nach Anspruch 2, das ein Balsam zur Haar- und Kopfhautpflege ist.

## Revendications

1. Composition cosmétique destinée à être incluse dans un produit de soins capillaires, comprenant une combinaison des composants suivants :
(a) un complexe minéral contenant une combinaison de sels minéraux cosmétiquement acceptables, dans laquelle ladite combinaison contient un sel de magnésium et un sel choisi parmi un sel de fer, un sel de calcium, un sel de cuivre, un sel de zinc ou toute combinaison de ceux-ci, dans laquelle ledit sel de magnésium est formé avec un acide aminé monobasique ou dibasique cosmétiquement acceptable, et ledit sel de fer, sel de calcium, sel de cuivre et/ou sel de zinc est formé avec un acide aldonique cosmétiquement acceptable,
(b) un complexe d'acides aminés contenant une combinaison de glycine et d'un dérivé N-trialkyle cosmétiquement acceptable de celle-ci, et
(c) un complexe d'extraction contenant une combinaison d'extraits eau-glycérine d'algues vertes *Chlorella* et d'algues rouges *Chondrus,*
dans laquelle les composants (a), (b) et (c) sont présents en le rapport de (0,5-10):(10-60):(10-400).

2. Produit cosmétique pour les soins capillaires, qui comprend une composition cosmétique selon la revendication 1 et un milieu cosmétiquement acceptable.

3. Produit selon la revendication 2, qui est un shampooing pour le soin capillaire et du cuir chevelu.

4. Produit selon la revendication 2, qui est un baume pour le soin capillaire et du cuir chevelu.
